Europäisches Patentamt

European Patent Office

Office européen des brevets

(11) Numéro de publication : **0 278 866 B1**

# FASCICULE DE BREVET EUROPEEN

(45) Date de publication du fascicule du brevet :
**18.12.91 Bulletin 91/51**

(51) Int. Cl.$^5$ : **A44B 18/00**, A61F 13/15

(21) Numéro de dépôt : **88400288.2**

(22) Date de dépôt : **09.02.88**

(54) Elément d'attache auto-agrippante notamment pour change complet à usage unique et procédé pour sa fabrication.

(30) Priorité : **10.02.87 FR 8701590**

(43) Date de publication de la demande :
**17.08.88 Bulletin 88/33**

(45) Mention de la délivrance du brevet :
**18.12.91 Bulletin 91/51**

(84) Etats contractants désignés :
**AT BE CH DE ES FR GB GR IT LI LU NL SE**

(56) Documents cités :
**EP-A- 0 235 014**
**WO-A-85/03205**
**WO-A-85/03625**
**FR-A- 2 198 712**
**GB-A- 2 146 230**
**US-A- 3 110 312**

(73) Titulaire : **KAYSERSBERG SA**
**Route de Lapoutroie**
**F-68240 Kaysersberg (FR)**

(72) Inventeur : **Pigneul, Raymond**
**2, rue des Vosges**
**F-68320 Durrenentzen (FR)**
Inventeur : **Ruppel, Rémy**
**7, rue des Sorbiers**
**F-68000 Horbourg (FR)**
Inventeur : **Brellmann, Jean**
**5, petite rue des Tanneurs**
**F-68000 Colmar (FR)**

(74) Mandataire : **David, Daniel**
**KAYSERSBERG 54, avenue Hoche**
**F-75008 Paris (FR)**

## Description

L'invention concerne le domaine des articles à usage unique, notamment les changes complets pour bébés ou adultes incontinents et porte sur un moyen de fixation auto-agrippant du type à boucles et à griffes. Elle vise en particulier l'élément pourvu de boucles.

La demande de brevet EP-A2-0235014 déposée par la demanderesse représentant un état de la technique selon l'article 54 (3) CBE et correspondant au document FR-A-2594650 — décrit un change complet dont les moyens d'attache, permettant l'ajustement du change autour de la taille, sont du type à boucles et à griffes. Un des éléments de chaque attache latérale est monté sur une patte solidaire d'un bord du matelas absorbant, sur la partie arrière du change, et l'élément complémentaire est fixé sur la face extérieure du matelas à la partie avant. On peut de la sorte, apis mise en place du change autour du bassin, enserrer la taille de l'utilisateur en joignant chacune des pattes latérales à sa partie complémentaire.

Dans cette demande de brevet, l'élément à griffes est de préférence monté sur la patte latérale et l'élément à boucles sur la face externe du matelas.

Afin de réduire les coûts de fabrication des articles à usage unique, on cherche à utiliser autant que possible des éléments eux-mêmes destines à être jetés après usage.

Il existe sur le marché des attaches auto-agrippantes dont l'élément à boucles est constitué d'un tissu pourvu sur l'une de ses faces de boucles relevées permettant l'accrochage des griffes, et réalisé suivant une armure lâche présentant une structure aérée.

Quand on veut fixer un élément de ce type sur la face extérieure du matelas, qui est de façon classique une feuille de polyéthylène, on utilise généralement une colle hot-melt, adhésive à température ambiante, entre l'élément et la feuille plastique. En machine de production, à cadence élevée, on applique l'adhésif sur l'une ou l'autre des faces devant venir en contact, puis on dépose l'élément sur la feuille en exerçant une pression pour assurer la liaison.

Ce mode de liaison couramment utilisé dans le domaine considéré n'est toutefois pas possible avec l'élément du type désiré, à cause de la fluidité de la substance adhésive. Elle traverse la structure ouverte du tissu au moment du pressage, et vient lier entre elles les boucles dont il est pourvu sur la face opposée. Ces boucles, une fois collées à plat, ne sont plus efficaces, il n'est plus possible d'accrocher les griffes de l'élément auto-agrippant complémentaire. L'attache est inutilisable.

L'invention a pour objet un élément pour attache auto-aggripante selon le préambule de la revendication 1, réalisé à partir d'un élément à boucles du type, connu en soi, mentionné ci-dessus, dont le tissu est à structure ouverte, il est caractérisé selon la partie caractérisante de la revendication 1 en ce que l'élément à boucles est recouvert au moins en partie d'une pellicule d'une résine synthétique de manière à permettre sa fixation sur un support au moyen d'une colle adhésive à froid.

L'expression structure ouverte est définie comme étant une structure dont l'ouverture entre les mailles est suffisante pour permettre le passage d'un fluide de viscosité correspondant à celle d'une colle hot-melt quand on exerce une pression sur l'élément, aux conditions prévues sur les machines utilisées dans le domaine des articles à usage unique.

Dans la pratique on reconnait qu'une structure est ouverte quand, après avoir enduit d'adhésif la face non bouclée de l'élément et l'avoir appliqué sur un support plan, on parvient à faire coller les boucles entre elles en pressant modérément avec le doigt sur la surface bouclée.

De préférence la pellicule est obtenue par enduction d'une colle déposée à chaud, dont la viscosité est suffisante pour éviter son passage au travers des mailles du tissu, et qui, une fois refroidie, n'a plus de tack résiduel. On rappelle que le "tack" est l'aptitude d'un adhésif à former immédiatement un joint de résistance mesurable.

Cette disposition présente de nombreux avantages :

— Après son refroidissement la pellicule forme écran. Au moment du pressage, la colle adhésive à froid est maintenue entre cette pellicule de la feuille de PE et assure leur liaison sans traverser le tissu. L'efficacité des boucles n'est pas altérée.

— La mise en oeuvre est très simple puisqu'il suffit, dans le cas d'une résine adhésive sans tack à froid, de réaliser un premier encollage avec cette résine, de refroidir la pellicule formée, puis d'appliquer l'élément au moyen de la colle adhésive à froid.

— La pellicule, selon la matière dont elle est constituée et son épaisseur, rigidifie le tissu et en amélioré la tenue. Cette propriété facilite le passage en machine du produit.

On connait par le brevet FR 2198712 un système de fermeture à pièce mâle et pièce femelle dont la seconde notamment est formée d'un substrat, tissé, pourvu d'une série de boucles se dressant à partir de son recto. Le verso de chaque pièce est recouvert d'une couche de résine synthétique adhésive, à bas point de fusion, afin de permettre la liaison de la pièce à un support par compression. Il est certain que ce mode de fixation au support ne s'adresse pas aux substrats à structure ouverte car la résine passerait au travers au moment du collage.

Conformément à un mode de réalisation préféré, on améliore encore la rigidité et la résistance de l'élément en lui adjoignant un support supplémentaire par l'intermédiaire de la pellicule de résine synthétique.

Ce support peut être un film de matière plastique tel que le polyéthylène, un textile tissé, un non-tissé, une grille synthétique ou bien un papier.

L'invention a également pour objet un procédé de fabrication d'un élément pour attache auto-agrippante, à partir d'un élément dont le tissu est à structure ouverte. Le procédé est caractérisé en ce qu'il consiste à solidariser audit élément une couche formant support par l'intermédiaire d'une pellicule de colle ne présentant pas de tack à froid.

L'invention est développée plus en détail dans la description non limitative qui suit de deux modes de réalisation, en regard des dessins annexés où :

— La figure 1, représente un change complet équipé d'attaches auto-agrippantes.

— La figure 2, représente un tissu à boucles dont la structure est ouverte.

— La figure 3, représente en coupe transversale, l'élément d'attache conforme à l'invention.

— La figure 4, représente en coupe transversale une deuxième forme de l'élément d'attache conforme à l'invention.

— La figure 5, est un schéma d'un dispositif permettant la réalisation de l'élément représenté à la figure 4.

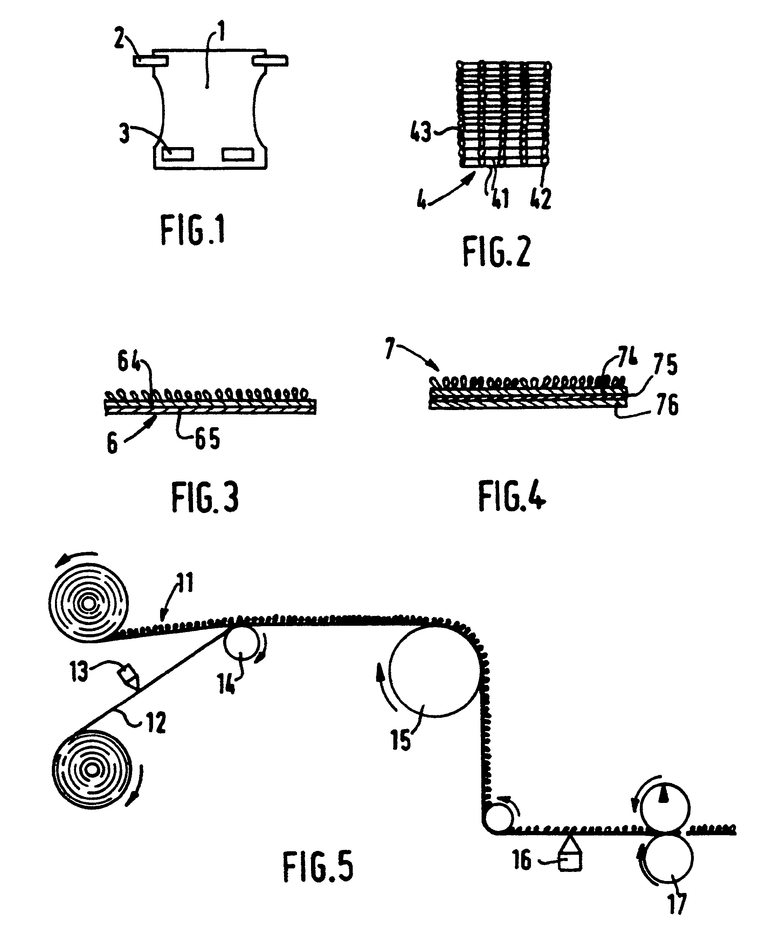

Sur la figure 1 est représenté un change complet à usage unique 1, équipé d'attaches auto-agrippantes. Le change est vu par sa face externe. Sur les pattes latérales 2 sont montées les éléments auto-agrippants à griffes, conformément à l'enseignement de la demande de brevet EP-A2-0235014 de la demanderesse. Mais ces éléments à griffes peuvent bien évidemment être quelconques. A l'autre extrémité du change, on a disposé les éléments à boucles, complémentaires de la fixation auto-agrippante. Ces éléments sont collés directement sur la face externe du change qui est, de façon classique, réalisée avec une feuille de polyéthylène. Mais l'invention est également applicable aux articles dont la face externe est un tissu quelconque, non-tissé par exemple.

Quand on utilise un élément à boucles dont le tissu est à mailles serrées, on peut sans difficulté assurer la liaison au moyen d'une couche visqueuse de colle adhésive à froid interposée entre la feuille externe du change et l'élément.

Toutefois quand on veut employer un élément à structure ouverte 3, telle que représentée schématiquement à la figure 2, la colle traverse le tissu au moment du pressage. Un élément de ce type est vendu par la Société LOUISON sous la référence 4090. Il s'agit d'une maille tricotée grattée, dite jetable, dont le fil de chaîne est en polyamide à 40 Drs et comporte 16 colonnes et 16 rangées au cm², le grammage est d'environ 90 g/m². On reconnaît sur la figure 2 les colonnes 41, les boucles 42 et les rangs 43. Pour une telle maille grattée jetable, avec cette grosseur du fil la densité de rangées et de colonnes au cm² peut varier entre 8 et 24.

L'espacement entre les colonnes d'une part et les mailles d'autre part est suffisamment important pour permettre le passage d'un fluide, même visqueux comme une colle adhésive à froid, par exemple la colle vendue par la Société BELIX sous la référence 72120, ou la colle vendue par la Société NATIONAL sous la référence DUROTACK 108.

Pour remédier à cet inconvénient, on emploie, figure 3, un élément 6 constitué de l'élément à boucles 64, du type précédent, sur lequel on a appliqué une pellicule 65 d'une résine synthétique. Cette pellicule peut recouvrir toute la surface ou bien être répartie en bandes parallèles ou en traits interrompus. On a utilisé une colle vendue par la Société BELIX sous la référence FERUWELD 204 que l'on peut déposer à l'aide d'un applicateur à buse ou à disque. La température de fusion de ce mélange est à 94°C et son point de blocage à 50°C ; à température ambiante elle ne présente donc plus de tack. Une autre colle qui convient également est la colle vendue par la Société FULLER sous la référence UNATACK 50. Cette pellicule 5 forme un écran entre le tissu 4 et l'adhésif.

Selon une autre forme de réalisation de l'invention, pour former l'élément à boucles 7, on dispose, conformément à la représentation de la figure 4, une couche supplémentaire 76 formant support sur la pellicule 75 de résine qui assure ainsi la liaison entre la couche 76 et l'élément à boucles 74. La couche 76 doit être en matériau à la fois souple et résistant, il peut s'agir d'un papier, d'un textile tissé, d'un film plastique, d'un non-tissé, ou d'une grille synthétique par exemple celle vendue sous la marque SCRINYL par la demanderesse et décrite dans le brevet FR-A-2195555. Cette couche supplémentaire apporte non seulement la résistance mais aussi une certaine rigidité au complexe ainsi obtenu, ce qui facilite son utilisation en machine.

Sur la figure 6, on a représenté schématiquement un dispositif permettant la réalisation des éléments représentés à la figure 4. Une bande de tissu à boucles 11 est déroulée à partir d'une bobine montée à rotation autour d'un axe. De la même façon, une bande 12 d'un matériau devant constituer la couche support de l'élément 7 est déroulée à partir d'une bobine montée sur un axe. Les deux bandes 11 et 12 se rejoignent sur un rouleau 14. La bande 12 est enduite, entre la bobine et le rouleau 14 d'une mince pellicule de colle hot-melt sans tack à froid, telle que la colle FERUWELD 204, au moyen d'une buse 13. Le complexe formé est posé ensuite autour d'un tambour, qui peut être réfrigéré par circulation d'eau, de manière à abaisser la température de l'adhésif jusqu'à ce qu'il ne présente plus de tack. En aval du tambour, on applique sur la bande 12 au moyen d'une buse 16 une colle hot-melt adhésive à froid et on découpe au moyen de couteaux 17 le complexe en éléments 7 prêts à l'emploi, qui peuvent être repris par des moyens appropriés non représentés pour être déposés sur la feuille formant la face externe du change.

Il est possible également d'enrouler le complexe en bobine après son refroidissement pour un stockage provisoire et permettre son emploi ultérieurement.

## Revendications

1. Elément (6, 7) pour attache auto-agrippante du type à boucles et à griffes, réalisé à partir d'un élément à boucles dont le tissu est à structure ouverte, caractérisé en ce que ledit élément à boucles (64, 74) comporte une pellicule (65, 75) d'une résine synthétique sur au moins une partie de sa surface non pourvue de boucles, la pellicule formant écran entre ladite colle adhésive à froid et le tissu de manière à permettre sa fixation sur un support au moyen d'une colle adhésive à froid.

2. Elément selon la revendication 1, caractérisé en ce que la résine (65, 75) est une colle ne présentant pas de tack à température ambiante.

3. Elément selon l'une des revendications précédentes, caractérisé en ce que la résine est disposée en traits continus parallèles.

4. Elément selon l'une des revendications précédentes, caractérisé en ce qu'il comporte une couche (76) supplémentaire formant support.

5. Elément selon la revendication précédente, caractérisé en ce que la couche supplémentaire (76) est choisie parmi les matériaux suivants : papier, nontissé, grille, tissé, film plastique.

6. Procédé pour réaliser un élément selon l'une des revendications 4 et 5 pour attache auto-agrippante du type à boucles et à griffes à partir d'un élément à boucles dont le tissu est à structure ouverte, caractérisé en ce qu'il consiste à dérouler une bande (12) destinée à former ladite couche formant support, à déposer sur la bande (12) une pellicule de colle à chaud ne présentant pas de tack à froid, à solidariser la bande (12) avec une bande de tissu à boucles, à refroidir le complexe formé et à découper le complexe en éléments individuels.

7. Procédé selon la revendication 6, caractérisé en ce que l'on dépose sur la couche support (12), un film de colle adhésive à froid.

8. Article à usage unique dont le système d'attache est du type à boucles et à griffes, caractérisé en ce que l'élément à boucles est du type défini à l'une quelconque des revendications précédentes.

9. Article à usage unique selon la revendication 8, caractérisé en ce que l'élément à boucles est solidarisé à une partie du change au moyen d'une colle adhésive à température ambiante.

## Patentansprüche

1. Element (6, 7) für einen selbsthaftenden Verschluß in Form eines Klettverschlusses mit Maschen und Häkchen, das aus einem Maschenelement hergestellt wird, dessen Gewebe eine offene Struktur hat, dadurch gekennzeichnet, daß das Maschenelement (64, 74) einen Film (65, 75) aus Kunstharz auf mindestens einer seiner nicht mit Maschen versehenen Oberfläche aufweist, wobei der Film einen Schirm zwischen dem Kaltkleber und dem Gewebe zur Befestigung an einem Träger mittels eines Kaltklebers bildet.

2. Element nach Anspruch 1, dadurch gekennzeichnet, daß der Harz (65, 75) ein Kleber ist, der bei Umgebungstemperatur nicht klebrig ist.

3. Element nach einem der vorhergehenden Ansprüche, dadurch gekennzeichnet, daß der Harz in parallelen kontinuierlichen Linien angeordnet ist.

4. Element nach einem der vorhergehenden Ansprüche, dadurch gekennzeichnet, daß es eine einen Träger bildende zusätzliche Schicht aufweist.

5. Element nach dem vorhergehenden Anspruch, dadurch gekennzeichnet, daß die zusätzliche Schicht (76) aus den folgenden Materialien ausgewählt wird : Papier, Vliesstoff, Gitterstoff, Gewebe, Kunststofffilm.

6. Verfahren zum Herstellen eines Elements nach einem der Ansprüche 4 und 5 für einen selbsthaftenden Verschluß in Form eines Klettverschlusses mit Maschen und Häkchen aus einem Maschenelement, dessen Gewebe eine offene Struktur hat, dadurch gekennzeichnet, daß ein Band (12), das die den Träger bildende Schicht bilden soll, abgewickelt wird, daß auf das Band (12) eine Schicht aus Heißkleber aufgetragen wird, der im Kaltzustand nicht klebrig ist, daß das Band (12) mit einem Band aus Maschengewebe verbunden wird, daß der entstandene Verbundstoff gekühlt und in einzelne Elemente zerschnitten wird.

7. Verfahren nach Anspruch 6, dadurch gekennzeichnet, daß auf die Trägerschicht (12) ein Film aus Kaltkleber aufgetragen wird.

8. Wegwerfartikel, dessen Verschlußsystem als Klettverschluß mit Maschen und Häkchen ausgebildet ist, dadurch gekennzeichnet, daß das Maschenelement als Maschenelement nach einem der vorhergehenden Ansprüche ausgebildet ist.

9. Wegwerfartikel nach Anspruch 8, dadurch gekennzeichnet, daß das Maschenelement mit einem beschnitt der Windel mit Hilfe eines Klebers bei Umgebungstemperatur verbunden ist.

## Claims

1. A member (6, 7) for a self-attaching connection of the loop and hook type, formed from a loop member whose fabric has an open structure, characterized in that the loop member (64, 74) comprises a film (65, 75) of a synthetic resin on at least part of its surface not provided with loops, the film forming a screen between the cold bonding glue and the fabric so as to allow it to be fastened to a support by a cold bonding glue.

2. A member as claimed in claim 1, characterized in that the resin (65, 75) is an adhesive having no tack at ambient temperature.

3. A member as claimed in one of the preceding claims, characterized in that the resin is disposed in parallel continuous lines.

4. A member as claimed in one of the preceding claims, characterized in that comprises a supplementary layer (76) forming a support.

5. A member as claimed in the preceding claim, characterized in that the supplementary layer (76) is selected from the following materials : paper, non-woven, grid, woven fabric, plastic film

6. A method for the production of a member as claimed in one of claims 4 and 5 for a self-attaching connection of the loop and hook type from a loop member whose fabric has an open structure, characterized in that it consists in unwinding a strip (12) adapted to form the layer forming a support, in depositing a film of hot adhesive having no tack when cold on the strip (12), in rigidly joining the strip (12) with a strip of loop fabric, in cooling the complex formed and in cutting the complex into individual members.

7. A method as claimed in claim 6, characterized in that a film of cold bonding glue is deposited on the support layer (12).

8. A disposable article whose connection system is of the type having loops and hooks, characterized in that the loop member is of the type defined in any one of the preceding claims.

9. A disposable article as claimed in claim 8, characterized in that the loop member is rigidly joined to a portion of the napkin by means of a glue adhesive at ambient temperature.

FIG.1

FIG.2

FIG.3

FIG.4

FIG.5